# EUROPEAN PATENT APPLICATION

(11) **EP 4 847 002 A1**
(43) Date of publication of application: **02.12.2026**
(21) Application number: 25857978.8
(22) Date of filing: 08.12.2025
(51) Int. Cl.: A61K 8/02, A61K 8/81, A61K 8/73, A61K 8/92, A61K 8/9761, A61K 8/9789, A61Q 5/02, A61Q 17/00, C11D 1/83, C11D 3/37, C11D 3/22, C11D 3/12, C11D 3/28, C11D 3/382, C11D 3/48, C11D 17/00, C11D 1/10, C11D 1/14, C11D 1/28, C11D 1/12, C11D 1/66

(54) **ANTIBACTERIAL CLEANING TABLET FOR WASHING AND CARE, METHOD FOR PREPARING SAME, METHOD FOR USING SAME, AND USE THEREOF**

(30) Priority: 02.01.2025 CN 202510004660
(71) Applicant: Guangzhou Joyson Cleaning Products Co., Ltd., Guangzhou, Guangdong 511458 (CN)
(72) Inventor: LI, Fenglei, Guangzhou Guangdong 511458 (CN); SUN, Min, Guangzhou Guangdong 511458 (CN); LUO, Jieer, Guangzhou Guangdong 511458 (CN); YAN, Jieting, Guangzhou Guangdong 511458 (CN); LI, Junli, Guangzhou Guangdong 511458 (CN)
(74) Representative: Patent 42
(86) International application number: PCT/CN2025/140725
(87) International publication number: WO 2026/144846

(57) **Abstract**

The present invention relates to the technical fields of washing, caring, and cleaning, and particularly relates to a bacteriostatic washing, caring, and cleaning sheet as well as a preparation method and use method thereof. In parts by mass, raw materials for preparation include: 10-30 parts of a film-forming agent, 10-40 parts of a surfactant, 1-10 parts of a filler, 0.5-6 parts of a chelating agent, 0.5-5 parts of a hair conditioner, 0.5-5 parts of a plant extract, 1-5 parts of an auxiliary agent, 0.1-1 part of a bacteriostatic agent, 0.1-1 part of an essence, and 10-100 parts of deionized water. The bacteriostatic washing, caring, and cleaning sheet of the present invention does not contain a sodium lauryl sulfate (SLS) surfactant, an ethoxylated compound surfactant, and a preservative, has the advantages of being mild in cleaning, high in solubility, and capable of inhibiting *Malassezia*.

## Description

### FIELD OF TECHNOLOGY

The present invention relates to the technical fields of washing, caring, and cleaning, and particularly relates to a bacteriostatic washing, caring, and cleaning sheet as well as a preparation method and use method thereof.

### BACKGROUND

Currently, shampoo products are mainly in the form of liquids or gels and are usually packaged in plastic bottles with most specifications of 100-1,000 mL, which are suitable for use by many individuals or families. However, the tourism and hotel industries have higher demands for small-dose shampoo products. Shampoo cleaning sheets are innovative shampoo products presented in the form of sheets, which are more portable and environmentally friendly compared with traditional liquid shampoos. During use, only one shampoo cleaning sheet needs to be placed in an appropriate amount of water for dissolution, or directly rubbed on wet hair to generate foam, followed by regular shampooing steps. The shampoo cleaning sheets are small in volume, easy to carry, and highly suitable for use in travel or business trips, thereby reducing the use of packaging materials, especially the use of plastic bottles, and helping to reduce environmental pollution. The shampoo cleaning sheets have advantages such as hygiene, precise dose, convenient portability, and environmental friendliness, thus having broad application prospects.

Since the shampoo cleaning sheets are obviously different from traditional shampoos in existence forms and use conditions, their formulations also have many technical problems that need to be addressed, for example, (1) solubility: the shampoo cleaning sheets need to be rapidly and effectively dissolved in water to rapidly generate sufficient foam for cleaning the hair, and to achieve this purpose, ingredients in the formulations need to ensure rapid dissolution without compromising a cleaning effect and mildness to the scalp; (2) stability: compared with the liquid shampoos, solid shampoo sheets need to maintain their physical forms unchanged within a long time, which means that the ingredients in the formulations need to have good stability and durability to prevent the occurrence of deterioration due to temperature changes or humidity impacts; (3) cleaning capability: the shampoo cleaning sheets need to possess effective oil removal and cleaning capabilities to meet the basic demand of consumers for hair cleaning, which requires that the cleaning ingredients in the formulations can be effectively presented in cleaning sheet carriers; and (4) moisturizing and care: in addition to basic cleaning functions, modern consumers also expect that the shampoo products can provide additional scalp caring and hair nourishing effects. Therefore, how to add effective nourishing ingredients within a limited space while maintaining other performances of the products unaffected is a challenge.

In the prior art, sulfate surfactants, represented by sodium fatty alcohol sulfate (mainly sodium alkyl sulfate, such as sodium lauryl sulfate, SLS) and sodium fatty alcohol polyether sulfate, are currently main surfactants used in washing, caring, and cleaning products. Chinese patent CN105769615A provides a portable shampoo sheet and a preparation method therefor. The shampoo sheet has a certain removal effect on various hair stains. However, on the one hand, this formulation contains a sodium laureth sulfate surfactant that easily produces dioxane, and an ethylene diamine tetraacetic acid tetrasodium salt used as a chelating agent also easily causes problems such as skin itching and allergies and is environmentally unfriendly. On the other hand, 15-20% of ordinary starch with poor water solubility is added as a filler. When used as a shampoo product, the starch may remain on the hair due to a small amount of water and a low water temperature.

Chinese patent CN105796385A provides an environmentally friendly ultra-concentrated shampoo sheet/granule and a preparation method thereof. In its formulation, sodium lauryl polyoxyethylene ether sulfate (AES) accounts for 58%, which may bring more dioxane into the shampoo sheet. Chinese patent CN106726634A provides a sheet-like shampoo product, in which sodium lauryl sulfate (SLS) accounts for 10-40%, and AES accounts for 1-10%. The product has excessively high cleaning capability, making it unsuitable for long-term use.

Therefore, the development of a bacteriostatic washing, caring, and cleaning sheet has a significant application prospect.

### SUMMARY

An objective of the present invention is to provide a bacteriostatic washing, caring, and cleaning sheet. The bacteriostatic washing, caring, and cleaning sheet of the present invention does not contain an SLS surfactant, an ethoxylated compound surfactant, and a preservative, has the advantages of being mild in cleaning, high in solubility, and capable of inhibiting *Malassezia*, and can be used for daily hair washing, hand washing, and underwear cleaning.

A first aspect of the present invention provides a bacteriostatic washing, caring, and cleaning sheet, where in parts by mass, raw materials for preparation include: 10-30 parts of a film-forming agent, 10-40 parts of a surfactant, 1-10 parts of a filler, 0.5-6 parts of a chelating agent, 0.5-5 parts of a hair conditioner, 0.5-5 parts of a plant extract, 1-5 parts of an auxiliary agent, 0.1-1 part of a bacteriostatic agent, 0.1-1 part of an essence, and 10-100 parts of deionized water.

In some embodiments, the film-forming agent includes a film-forming agent A and a film-forming agent B at a mass ratio of (2-20):1; and the film-forming agent A is polyvinyl alcohol, and the film-forming agent B is selected from at least one of starch acetate and hydroxyethyl cellulose.

The polyvinyl alcohol, being rich in hydroxyl, has strong affinity with the starch acetate and the hydroxyethyl cellulose. The inventor has found that physical blending of the polyvinyl alcohol with the starch acetate and the hydroxyethyl cellulose within a certain ratio range can improve the moisture resistance of the cleaning sheet during daily storage, and can reduce the problem of poor water solubility of a composite film of the polyvinyl alcohol and other bio-based polymers when combined with the specific surfactant, thereby enabling rapid dissolution during use.

The starch acetate is generated by an esterification reaction of starch under the action of acetic acid, and has high safety and biodegradability. Compared with ordinary starch, the starch acetate has higher water dispersibility, heat resistance, and film-forming property, can improve the moisture resistance of products when used as a film-forming agent for shampoo sheets, and additionally, can be rapidly and uniformly stirred during a slurry preparation process due to the higher water dispersibility, thereby increasing production efficiency.

In some embodiments, the surfactant includes an amino acid surfactant, an anionic surfactant, and a nonionic surfactant at a mass ratio of (10-35):(3-32):(1-10).

In some embodiments, the amino acid surfactant includes at least one of lauroyl glutamate, lauroyl sarcosinate, myristoyl glutamate, palmitoyl glutamate, and cocoyl glycinate.

The amino acid surfactant has characteristics such as mild performance, low irritation, and good biodegradability, but tends to affect the viscosity of a product. The inventor has found that when the amino acid surfactant and the anionic surfactant are mixed, an electrostatic interaction between the two may lead to an increase in intermolecular distance, thereby reducing the viscosity of the system. Compared with liquid shampoo products that can increase the product viscosity by adding a thickener, sheet-like shampoo products require a slurry to have appropriate viscosity. If the viscosity is too low, molding cannot be achieved, or a molding speed is low, leading to excessive dryness, brittleness, and frangibility of the products; and if the viscosity is too high, a loading speed during a sheet production process is low, and slurry tailing leads to the inability to form sheets.

In some embodiments, the anionic surfactant includes at least two of α-olefin sulfonate, fatty acid methyl ester sulfonate, and methyl lauroyl taurate.

In some embodiments, the nonionic surfactant includes at least one of alkyl glucoside, maltoside, and xylityl glucoside.

In some embodiments, the filler includes at least one of kaolin, bentonite, porous starch, cyclodextrin, and maltodextrin.

The specific filler selected in the present invention not only can assist in the formation of the shampoo sheet, but also can adsorb active substances such as the hair conditioner and the plant extract, thus protecting the active substances during a sheet production process. Furthermore, during use, after the active substances are released, the filler can adsorb oil on the scalp and the hair, thus playing a role in cleaning oil and dirt on the follicle and the scalp.

In some embodiments, the hair conditioner includes at least one of allantoin, shea butter, coconut oil, hydrolyzed soy protein, hydrolyzed corn starch, and hydrolyzed wheat protein.

In some embodiments, the plant extract includes at least one of tea tree oil, a *Platycladus orientalis* leaf extract, a *Camellia oleifera Abel.* cake extract, and a *Sapindus mukorossi* extract.

The natural plant extract has the characteristics of being green, non-toxic, mild, safe, easily degradable, and the like. The specific tea tree oil, a perilla leaf, a *Camellia oleifera Abel.* cake, and *Sapindus mukorossi* selected in the present invention are rich in active ingredients such as saponins, volatile oils, and fatty acids, enabling the product to have multiple effects, such as anti-inflammatory, bacteriostatic, bactericidal, anti-itching, and anti-dandruff effects, while maintaining cleaning capability.

In some embodiments, the bacteriostatic agent includes at least one of colloidal sulfur, selenium disulfide, and piroctone olamine.

In some embodiments, the chelating agent includes at least one of citric acid and its salts, methylglycine diacetic acid and its salts, and glutamic acid diacetic acid and its salts.

In some embodiments, the auxiliary agent includes dimethylsiloxane and a polyol, where the polyol includes at least one of glycerol, propylene glycol, butanediol, hexanediol, octanediol, sorbitol, and mannitol.

A second aspect of the present invention provides a method for preparing the bacteriostatic washing, caring, and cleaning sheet, which includes the following steps:
S1. uniformly mixing the filler with the hair conditioner, the plant extract, and a half mass of the deionized water at 25-35°C to obtain a composition; and
S2. mixing the film-forming agent with the remaining deionized water, heating up to 75-85°C, stirring at a constant temperature until the material is swollen and fully dissolved, adding the surfactant and the chelating agent, stirring for uniform dissolution, then cooling to 45-60°C, adding the composition, stirring uniformly to obtain a slurry, oven-drying, molding for sheet preparation, and slicing to obtain the bacteriostatic washing, caring, and cleaning sheet.

A third aspect of the present invention provides use of the bacteriostatic washing, caring, and cleaning sheet, where the bacteriostatic washing, caring, and cleaning sheet is used for cleaning hair, hands, and clothing.

Compared with the prior art, the present invention has the following beneficial effects.

Traditional anti-dandruff components, such as sulfur, selenium disulfide, and piroctone olamine, have poor water solubility, precipitation resistance, and dispersibility, and tend to sink and stratify in shampoo liquids, resulting in uneven distribution of anti-dandruff agents and excessively high local concentrations. Currently, a large amount of a thickener is usually added to shampoo liquids to reduce fluidity to improve the above problems. However, as the storage time prolongs, the anti-dandruff components eventually sink to the bottom. The innovative washing and caring sheet product of the present invention can fundamentally solve the above problems.

### BRIEF DESCRIPTION OF THE DRAWINGS

FIGs. 1-2 show experimental results of a volunteer trial test.
FIG. 3 is a bacteriostatic test report.

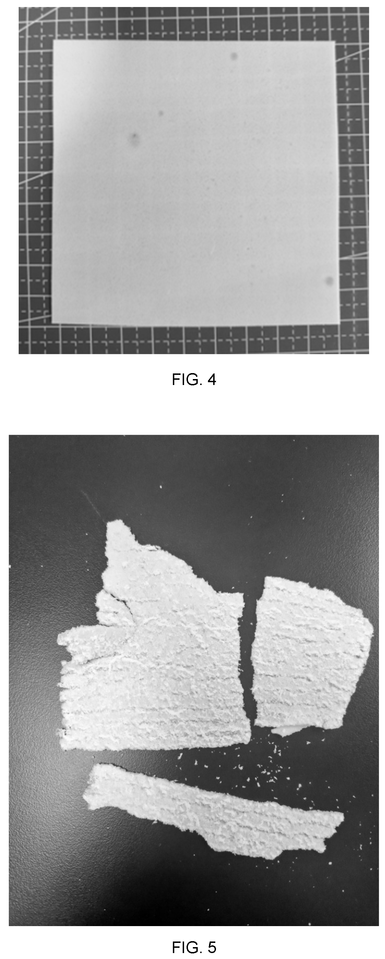

FIG. 4 is a physical image of a product.
FIG. 5 is an image of a product of Comparative Example 2.

### DESCRIPTION OF THE EMBODIMENTS

The technical solutions in the examples of the present invention are described clearly and completely below. Obviously, the described examples are only a part of the examples of the present invention, rather than all of the examples. Based on the examples of the present invention, all other examples obtained by those of ordinary skill in the art without exerting creative labor are within the scope of protection of the present invention.

### Example 1

Examples and comparative examples provide a bacteriostatic washing, caring, and cleaning sheet. In parts by mass, raw materials for preparation are shown in Table 1. The sheet includes the following steps:
S1. uniformly mixing a filler with a hair conditioner, a plant extract, and a half mass of deionized water at 30°C to obtain a composition; and
S2. mixing a film-forming agent with the remaining deionized water, heating up to 80°C, stirring at a constant temperature until the material swells and is fully dissolved, adding a surfactant and a chelating agent, stirring for uniform dissolution, then cooling to 50°C, adding the composition, stirring uniformly to obtain a slurry, oven-drying, molding for sheet preparation, and slicing to obtain the bacteriostatic washing, caring, and cleaning sheet.

**Table 1**

| | | Example 1 | Example 2 | Comparative Example 1 | Comparative Example 2 | Comparative Example 3 | Comparative Example 4 |
|---|---|---|---|---|---|---|---|
| Film-forming agent A | Polyvinyl alcohol | 19 | 18 | 19 | 19 | 19 | 19 |
| Film-forming agent B | Corn starch acetate | 1 | / | 1 | 1 | 1 | 1 |
| | Wheat starch acetate | / | 4 | / | / | / | / |
| | Hydroxye thyl cellulose | 0.5 | / | 0.5 | 0.5 | 0.5 | 0.5 |
| Amino acid surfactant | Sodium lauroyl sarcosin ate | 10 | / | 10 | 10 | 4.4 | 2.2 |
| | Sodium lauroyl glutamate | / | 12.5 | / | / | / | / |
| | Sodium palmitoyl glutamate | / | 2.5 | / | / | / | / |
| | Sodium myristoyl glutamate | 2.5 | / | 2.5 | 2.5 | 1.1 | 0.55 |
| | Sodium cocoyl glycinate | 2.5 | / | 2.5 | 2.5 | 1.1 | 0.55 |
| Anionic surfactant | Sodium α-olefin sulfonate | 11 | 5 | / | 11 | 6.6 | 9.9 |
| | Sodium fatty acid methyl ester sulfonate | / | 2.1 | / | / | / | / |
| Nonionic surfactant | Alkyl glucoside | 2 | / | 2 | / | 2.2 | 2.2 |
| | Maltoside | 1 | / | 1 | / | 1.1 | 1.1 |
| | Lauryl glucoside | / | 1.5 | / | / | / | / |
| | Xylityl glucoside | / | 0.4 | / | / | / | / |
| Filler | Kaolin | 2 | 0.5 | 13 | 5 | 2 | 2 |
| | Porous starch | 0.5 | 1.5 | 0.5 | 0.5 | 0.5 | 0.5 |
| | Maltodex trin | 1.5 | / | 1.5 | 1.5 | 1.5 | 1.5 |
| Hair condition er | Allantoin | 0.1 | / | 0.1 | 0.1 | 0.1 | 0.1 |
| | Shea butter | 0.2 | / | 0.2 | 0.2 | 0.2 | 0.2 |
| | Hydrolyz ed soy protein | / | 0.2 | / | / | / | / |
| | Coconut oil | / | 0.2 | / | / | / | / |
| Plant extract | Tea tree oil | 0.2 | 0.2 | 0.2 | 0.2 | 0.2 | 0.2 |
| | *Camellia oleifera Abel.* cake extract | 0.4 | 0.6 | 0.4 | 0.4 | 0.4 | 0.4 |
| | *Sapindus mukoros si* extract | 0.6 | 0.8 | 0.6 | 0.6 | 0.6 | 0.6 |
| Bacterios tatic agent | Colloidal sulfur | 0.2 | 0.3 | 0.2 | 0.2 | 0.2 | 0.2 |
| Chelating agent | Citric acid | 0.15 | / | 0.15 | 0.15 | 0.15 | 0.15 |
| | Sodium citrate | / | 1.3 | / | / | / | / |
| Auxiliary agent | Trisodiu m methylgly cine diacetate | 1.3 | 0.1 | 1.3 | 1.3 | 1.3 | 1.3 |
| | Dimethyl siloxane | 0.2 | / | 0.2 | 0.2 | 0.2 | 0.2 |
| | Butanedi ol | 2.5 | / | 2.5 | 2.5 | 2.5 | 2.5 |
| | Glycerol | / | 3 | / | / | / | / |
| | Essence | 0.2 | 0.2 | 0.2 | 0.2 | 0.2 | 0.2 |
| | Deionized water | 40.45 | 36.9 | 40.45 | 40.45 | 40.45 | 40.45 |

### Performance tests

The following tests were conducted on the bacteriostatic washing, caring, and cleaning sheet prepared in examples and comparative examples.

### (1) Bacteriostatic test

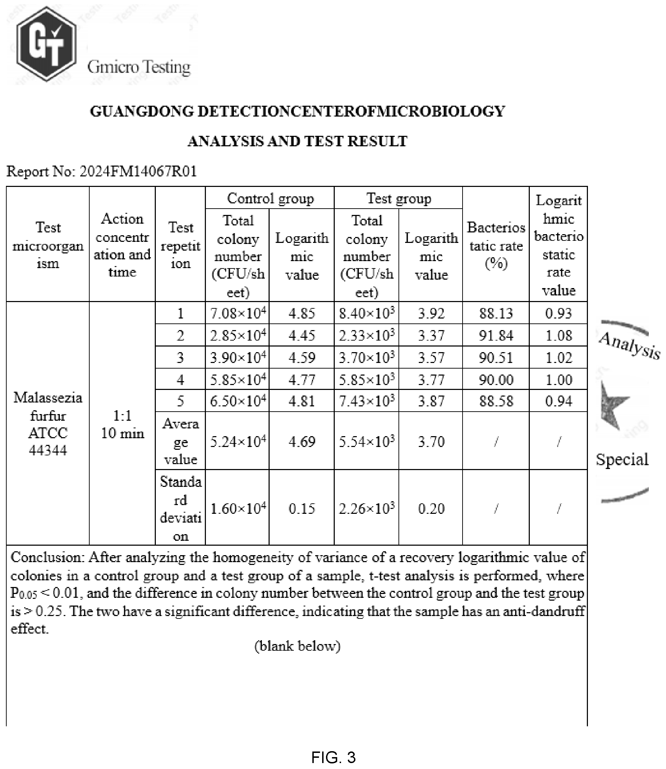

The Guangdong Detection Center of Microbiology was commissioned to test an inhibitory effect of the bacteriostatic washing, caring, and cleaning sheet (sample) prepared in Example 1 on the growth of *Malassezia furfur* for an action time of 10 minutes at an action concentration of 1:1 according to a test method for anti-dandruff efficacy of anti-dandruff products in T/GDCA 010-2022-7 "Carrier Immersion Antimicrobial Test". Experimental results are shown in Table 2 (FIG. 3 is a third-party test report).

**Table 2**

| Experiment repetition | Control group | | Example 1 | | Bacteriosta tic rate (%) | Logarithmi c bacteriost atic rate value |
|---|---|---|---|---|---|---|
| | Total colony number (CFU/sheet) | Logarith mic value | Total colony number (CFU/sheet) | Logarith mic value | | |
| 1 | 7.08×10⁴ | 4.85 | 8.40×10³ | 3.92 | 88.13 | 0.93 |
| 2 | 2.85×10⁴ | 4.45 | 2.33×10³ | 3.37 | 91.84 | 1.08 |
| 3 | 3.90×10⁴ | 4.59 | 3.70×10³ | 3.57 | 90.51 | 1.02 |
| 4 | 5.85×10⁴ | 4.77 | 5.85×10³ | 3.77 | 90.00 | 1.00 |
| 5 | 6.50×10⁴ | 4.81 | 7.43×10³ | 3.87 | 88.58 | 0.94 |
| Average value | 5.24×10⁴ | 4.69 | 5.54×10³ | 3.7 | / | / |
| Standard deviation | 1.60×10⁴ | 0.15 | 2.26×10³ | 0.2 | / | / |

Conclusion: After analyzing the homogeneity of variance of a recovery logarithmic value of colonies in a control group and a test group of the sample, t-test analysis is performed, where P0.05 < 0.01, and the difference in colony number between the control group and the test group is > 0.25. The two have a significant difference, indicating that the sample has an anti-dandruff effect.

### (2) Volunteer trial test

20 volunteers with heavy dandruff and prone to scalp itching were selected, including 10 males and 10 females. Within a trial period of 14 days, the males used the sample once daily at 1 time per day, and the females used the sample 1 time every 2 days. After hair washing and blow-drying, changes in dandruff were observed after 12 hours using a scalp tester (Model: HDN-XM01).

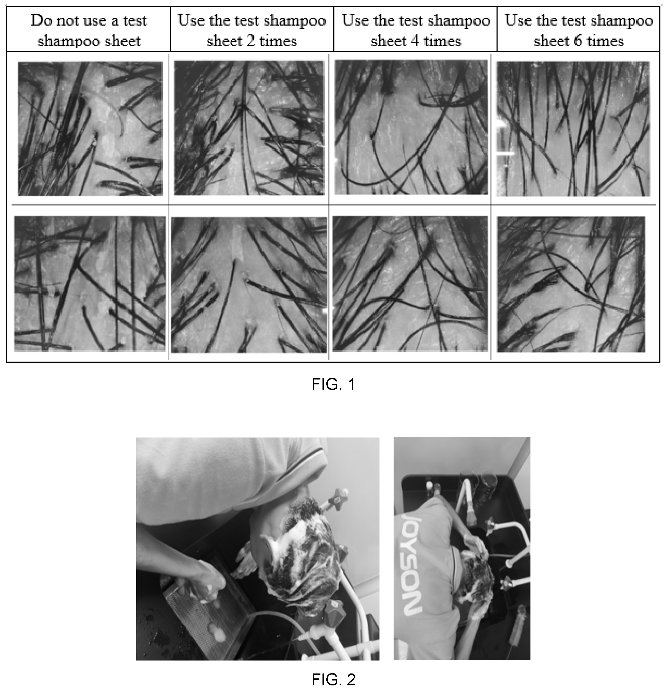

Experimental results are shown in FIGs. 1-2. Sensory report results of the volunteers are shown in Table 4.

### (3) Viscosity test

Test method: According to GB/T 5561-2012 "Surfactants - Determination of viscosity and flow properties using a rotational viscometer", the viscosity of a slurry was measured at 60±0.5°C using needle #4 at a rotation speed of 6. Results are shown in Table 3.

**Table 3**

| | Viscosity at 45°C (mPa•S) | Forming stability |
|---|---|---|
| Example 1 | 16612 | Form a sheet |
| Example 2 | 15496 | Form a sheet |
| Comparative Example 1 | 7966 | Too thin, unable to form a sheet |
| Comparative Example 2 | 9083 | Brittle, unable to form a sheet |
| Comparative Example 3 | 7258 | Too thin, unable to form a sheet |
| Comparative Example 4 | 35158 | Too thick, unable to form a sheet |

### (4) Volunteer sensory report

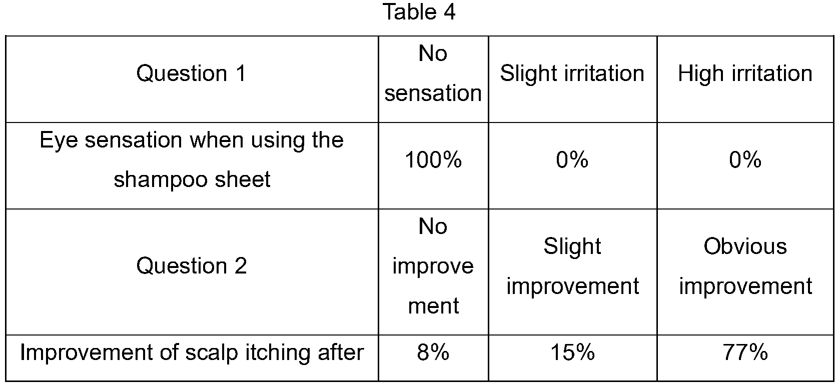

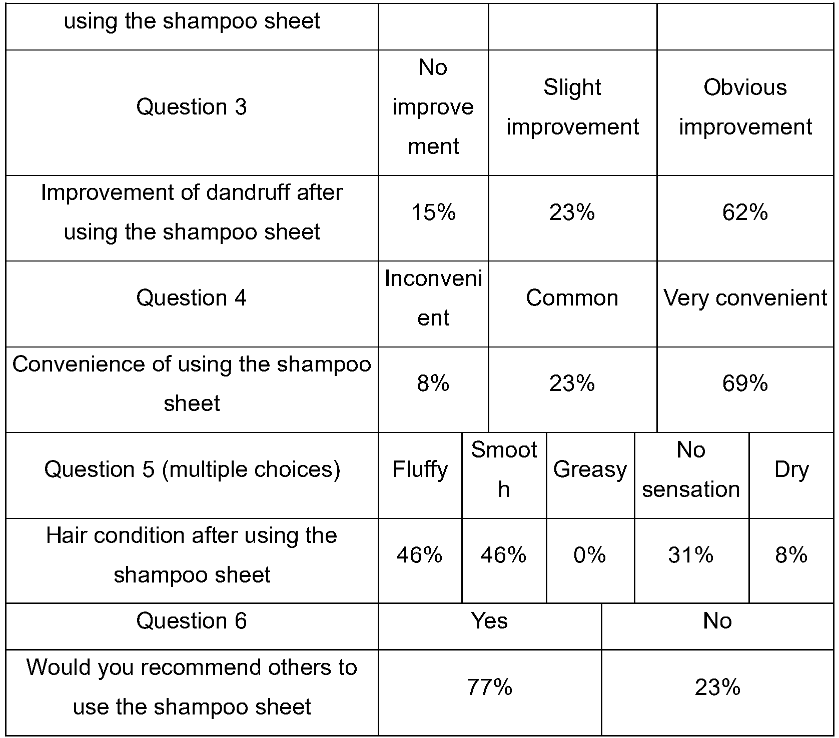

The descriptions above are preferred embodiments of the present invention. It should be noted that for those of ordinary skill in the art, various improvements and modifications can be made without departing from the principles of the present invention, and these improvements and modifications should also be regarded as falling within the scope of protection of the present invention.

## Claims

1. A bacteriostatic washing, caring, and cleaning sheet, **characterized in that** in parts by mass, raw materials for preparation comprise: 10-30 parts of a film-forming agent, 10-40 parts of a surfactant, 1-10 parts of a filler, 0.5-6 parts of a chelating agent, 0.5-5 parts of a hair conditioner, 0.5-5 parts of a plant extract, 1-5 parts of an auxiliary agent, 0.1-1 part of a bacteriostatic agent, 0.1-1 part of an essence, and 10-100 parts of deionized water.

2. The bacteriostatic washing, caring, and cleaning sheet according to claim 1, **characterized in that** the film-forming agent comprises a film-forming agent A and a film-forming agent B at a mass ratio of (2-20):1; and the film-forming agent A is polyvinyl alcohol, and the film-forming agent B is selected from at least one of starch acetate and hydroxyethyl cellulose.

3. The bacteriostatic washing, caring, and cleaning sheet according to claim 1, **characterized in that** the surfactant comprises an amino acid surfactant, an anionic surfactant, and a nonionic surfactant at a mass ratio of (10-35):(3-32):(1-10).

4. The bacteriostatic washing, caring, and cleaning sheet according to claim 3, **characterized in that** the amino acid surfactant comprises at least one of lauroyl glutamate, lauroyl sarcosinate, myristoyl glutamate, palmitoyl glutamate, and cocoyl glycinate.

5. The bacteriostatic washing, caring, and cleaning sheet according to claim 3, **characterized in that** the anionic surfactant comprises at least two of α-olefin sulfonate, fatty acid methyl ester sulfonate, and methyl lauroyl taurate.

6. The bacteriostatic washing, caring, and cleaning sheet according to claim 3, **characterized in that** the nonionic surfactant comprises at least one of alkyl glucoside, maltoside, and xylityl glucoside.

7. The bacteriostatic washing, caring, and cleaning sheet according to claim 1, **characterized in that** the filler comprises at least one of kaolin, bentonite, porous starch, cyclodextrin, and maltodextrin.

8. The bacteriostatic washing, caring, and cleaning sheet according to claim 1, **characterized in that** the hair conditioner comprises at least one of allantoin, shea butter, coconut oil, hydrolyzed soy protein, hydrolyzed corn starch, and hydrolyzed wheat protein, and/or the bacteriostatic agent comprises at least one of colloidal sulfur, selenium disulfide, and piroctone olamine.

9. A method for preparing the bacteriostatic washing, caring, and cleaning sheet according to any one of claims 1-8, **characterized by** comprising the following steps:
S1. uniformly mixing the filler with the hair conditioner, the plant extract, and a half mass of the deionized water at 25-35°C to obtain a composition; and
S2. mixing the film-forming agent with the remaining deionized water, heating up to 75-85°C, stirring at a constant temperature until the material is swollen and fully dissolved, adding the surfactant and the chelating agent, stirring for uniform dissolution, then cooling to 45-60°C, adding the composition, stirring uniformly to obtain a slurry, oven-drying, molding for sheet preparation, and slicing to obtain the bacteriostatic washing, caring, and cleaning sheet.

10. Use of the bacteriostatic washing, caring, and cleaning sheet according to any one of claims 1-8, **characterized in that** the bacteriostatic washing, caring, and cleaning sheet is used for cleaning hair, hands, and clothing.
